# FASCICULE DE BREVET EUROPEEN

(11) **EP 0 694 301 B1**
(45) Date de publication et mention de la délivrance du brevet: **13.11.1996**
(21) Numéro de dépôt: 95401477.5
(22) Date de dépôt: 21.06.1995
(51) Int. Cl.: A61K 31/38, A61K 31/195, A61K 31/19

(54) **Mélange synergétique d'au moins un ligand spécifique des RXRs et au moins un ligand spécifique de RAR-alpha**
Synergistische Mischung mindestens eines RXR-spezifischen Liganden mit mindestenseinem RAR-Alpha spezifischen Liganden
Synergistic combination containing at least a RXR specific ligand and a RAR alpha specific ligand

(30) Priorité: 27.07.1994 FR 9409307
(43) Date de publication de la demande: 31.01.1996
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Michel, Serge, F-06330 Roquefort Les Pins (FR); Lenoir-Viale, Marie-Cécile, F-06560 Valbonne (FR); Cathelineau, Christine, F-06560 Valbonne (FR); Demarchez, Michel, F-06620 Le Bar Sur Loup (FR)
(74) Mandataire: Tezier Herman, Béatrice

(56) Documents cités:
- WO-A-93/03713
- WO-A-93/21146
- PROC ANNU MEET AM ASSOC CANCER RES;33:A565-6 15-07-1992, SPORN MB ET AL 'MOLECULAR AND CELLULAR BASIS FOR THE USE OF RETINOIDS IN CHEMOPREVENTION (MEETING ABSTRACT)'
- BLOOD, 84 (2). 1994. 446-452., DAWSON M I ET AL 'Myeloid differentiation mediated through retinoic acid receptor-retinoic X receptor (RXR) not RXR-RXR pathway'
- NUCLEIC ACIDS RES, MAR 11 1993, 21 (5) P1231-7, ENGLAND, SCHRADER M ET AL 'RXR-dependent and RXR-independent transactivation by retinoic acid receptors.'
- NEW ENGL. J. MED., 1993, 329/3 (177-189), USA, WARRELL R.P. JR. ET AL 'Acute promyelocytic leukemia'

## Description

L'invention concerne de nouvelles compositions, notamment pharmaceutiques, comprenant des associations synergétiques entre d'une part au moins un ligand présentant une activité sélective pour les recepteurs nucléaires de type RXRs et, d'autre part, au moins un ligand présentant une activité sélective pour les récepteurs nucléaires de type RAR-α, ainsi que l'utilisation desdites compositions comme modulateurs de la prolifération et/ou de la différenciation d'un type cellulaire particulier (cellules HL-60), en particulier pour le traitement par voie systémique de la leucémie promyélocytaire aigüe.

On sait que l'acide rétinoïque est un puissant modulateur (i.e. un inhibiteur ou, au contraire, un stimulateur, selon la nature des cellules traitées) de la différenciation et de la prolifération de nombreux types cellulaires normaux ou transformés. Par exemple, il inhibe la différenciation des cellules épithéliales, tels que les kératinocytes de l'épiderme. Il inhibe aussi la prolifération de nombreuses cellules transformées telles que les cellules de mélanomes. Ces effets sur la prolifération et la différenciation peuvent affecter simultanément un même type cellulaire, comme cela est par exemple le cas pour les cellules promyélocytaires humaines référencées HL-60 ; ainsi, il est connu que la prolifération de ces cellules est inhibée par l'acide rétinoïque et, dans le même temps, que leur différenciation en granulocytes est augmentée.

Les cellules HL-60 ci-dessus sont particulièrement intéressantes et importantes, compte tenu principalement du fait qu'elles sont analogues aux cellules promyélocytaires des patients qui sont atteints de leucémie promyélocytaire aiguë. Ces patients sont actuellement traités avec l'acide rétinoïque, ce traitement entrainant, au moins dans un premier temps, une rémission notable de la maladie. Toutefois, on observe chez les patients ainsi traités, un phénomène de résistance ou d'accoutumance à l'égard de l'acide rétinoïque, qui nuit à terme à l'efficacité du traitement.

On sait, d'une manière générale, que l'acide rétinoïque tout-*trans* (all-*trans* retinoic acid) agit sur la différenciation et la prolifération des cellules en interagissant avec des récepteurs nucléaires ou RARs (Retinoic Acid Receptors) contenus dans le noyau cellulaire. Il existe, à ce jour, trois sous-types identifiés de récepteurs RAR appellés respectivement RAR-α, RAR-β et RAR-γ. Ces récepteurs, après fixation du ligand (i.e. de l'acide rétinoïque), interagissent avec la région promotrice de gènes régulés par l'acide rétinoïque au niveau d'éléments de réponses spécifiques. Pour se fixer sur les éléments de réponse, les RARs s'hétérodimèrisent avec un autre type de récepteurs connus sous le nom de RXRs. Le ligand naturel des RXRs est l'acide 9-*cis*-rétinoïque. Comme pour l'acide rétinoïque tout-*trans*, il est connu que l'acide 9-*cis*-rétinoïque est capable d'une part d'inhiber la prolifération des cellules HL-60 susmentionnées et, d'autre part, de stimuler leur différenciation en granulocytes. Les RXRs sont considérés comme des "master regulatory proteins" car ils interagissent avec d'autres membres de la superfamille des récepteurs stéroïdiens tels que le récepteur de la vitamine D3 (VDR) ou le récepteur de la triiodothyroxine (TR). De plus, les RXRs peuvent interagir avec des éléments de réponse spécifiques sous forme d'homodimères.

De nombreux analogues structuraux synthétiques de l'acide rétinoïque ou de l'acide 9-*cis*-rétinoïque, couramment dénommés "rétinoïdes", ont été décrits à ce jour dans la littérature. Certaines de ces molécules sont capables de se fixer et d'activer spécifiquement les RARs ou, au contraire, les RXRs. De plus, certains analogues peuvent se fixer et activer un sous-type de récepteur RAR (α, β ou γ) particulier. D'autres analogues, enfin, ne présentent aucune activité sélective particulière vis à vis de ces différents récepteurs. A cet égard, et par exemple, l'acide 9-*cis* rétinoïque active à la fois les RARs et les RXRs, sans sélectivité notable pour l'un ou l'autre de ces récepteurs (ligand non spécifique), alors que l'acide rétinoïque tout-*trans* active, quant à lui, sélectivement les RARs (ligand spécifique RARs), tous sous-types confondus. D'une manière générale et qualitativement, une substance donnée (ou ligand) est dite sélective vis à vis d'une famille de récepteurs donnée (respectivement vis à vis d'un récepteur particulier de cette famille) lorsque ladite substance présente une forte affinité pour l'ensemble des récepteurs de cette famille (respectivement pour le récepteur particulier de cette famille) et qu'elle présente par ailleurs une faible affinité pour tous les récepteurs de toute autre famille (respectivement pour tous les autres récepteurs, de cette même famille ou pas). Quantitavement, I'affinité se mesure au moyen des techniques de binding classiques (valeurs Kd) et l'on admet de manière générale que toute substance qui, à l'égard d'un premier recepteur donné, présente un Kd au moins 10 fois inférieur, et de préférence au moins 15 fois inférieur, au Kd qu'elle présente à l'égard d'un deuxième récepteur donné, peut être qualifiée de substance selective vis à vis de ce premier récepteur par rapport à ce deuxième récepteur. L'évaluation du caractère sélectif ou non d'une substance donnée à l'égard d'un récepteur donné est classiquement réalisée au moyen de tests biologiques *in vitro* connus en soi, comme cela sera rappelé ci-après.

La Demanderesse vient maintenant de découvrir que l'association, nouvelle en soi, et notamment à titre de médicament, entre (i) un rétinoïde spécifique d'un sous-type de récepteur particulier, à savoir un récepteur RAR-α, et (ii) un rétinoïde spécifique des RXRs, permettait de moduler de manière tout à fait remarquable la prolifération d'une part (qui est ici inhibée) et la différenciation d'autre part (qui est ici augmentée) des cellules HL-60 décrites précédemment. Ce résultat est d'autant plus inattendu et surprenant qu'une substance spécifique des RXRs, lorsqu'elle est utilisée seule, ne présente aucune, ou substantiellement aucune, activité propre vis à vis de ces mêmes cellules, et qu'un rétinoïde spécifique des RARs (tous sous-types confondus) est également inactif, toujours lorsqu'il est utilisé seul. Par ailleurs, l'association d'un rétinoïde spécifique de RAR-β ou de RAR-γ avec un rétinoïde spécifique des RXRs ne montre, quant à elle, aucune activité.

Cette découverte est à la base de la présente invention.

L'association conforme à l'invention, à savoir entre un rétinoïde spécifique de RAR-α et un rétinoïde spécifique des RXRs trouve naturellement, compte-tenu des activités remarquables qu'elle présente vis-à-vis des cellules HL-60, une application privilégiée dans le traitement par voie systémique des patients atteints de leucémie promyélocytaire aiguë.

Ainsi, dans l'un de ses premiers aspects, la présente invention a pour objet de nouvelles compositions, notamment médicamenteuses, qui sont essentiellement caractérisées par le fait qu'elles comprennent, dans un support physiologiquement acceptable, au moins un premier ligand présentant une activité sélective pour les recepteurs RXRs et au moins un deuxième ligand présentant une activité sélective pour le récepteur RAR-α.

L'invention a également pour objet l'utilisation des compositions ci-dessus à titre de, ou pour la fabrication de, compositions pharmaceutiques et/ou dermatologiques (médicaments) plus particulièrement destinées à moduler la prolifération et/ou la différenciation d'un système cellulaire de type HL-60, notamment pour le traitement systémique de la leucémie promyélocytaire aigüe.

D'une manière générale, on notera que les doses d'actifs à mettre en oeuvre en association pour obtenir l'effet recherché restent, selon l'invention, toujours très faibles, ce qui constitue un avantage important lorsqu'il s'agit de faire face à des problèmes de tolérance ou d'effets secondaires indésirables susceptibles d'apparaitre au sein des organismes à traiter ou en cours de traitement.

D'autres caractéristiques, aspects, objets et avantages de l'invention apparaitront encore plus clairement à la lecture de la description et des figures qui vont suivre, ainsi que des divers exemple concrets, mais nullement limitatifs, destinés à l'illustrer.

### Description des figures (la nature des composés 1 et 4 ci-dessous est donnée plus loin dans la description):

**Figure 1:** Effet de l'association du composé 1 spécifique de RAR-α et du composé 4 spécifique des RXRs sur la prolifération des cellules HL-60.

**Figure 2:** Effet de l'association du composé 1 spécifique de RAR-α et du composé 4 spécifique des RXRs sur la différenciation des cellules HL-60.

**Figure 3:** Effet de l'association du composé 3 (à savoir l'acide 6-[3-(1-adamantyl)-4-hydroxyphényl]-2-naphtoïque, utilisé ci-après dans les exemples) spécifique de RAR-γ et du composé 4 spécifique des RXRs sur la prolifération des cellules HL-60.

**Figure 4:** Effet de l'association du composé 3 spécifique de RAR-γ et du composé 4 spécifique des RXRs sur la différenciation des cellules HL-60.

Dans ce qui suit, on entend par voie topique, toute technique d'administration d'un produit par application directe de ce dernier sur une partie superficielle (ou externe) du corps, telle que la peau, et par voie systémique, toute technique d'administration d'un produit par une voie autre que topique, par exemple orale et/ou parentérale.

La présente invention a donc pour objet, entre autres, de nouvelles compositions médicamenteuses destinées notamment au traitement de l'affection susmentionnée, et qui sont caractérisées par le fait qu'elles comprennent, dans un support physiologiquement acceptable et compatible avec le mode d'administration retenu pour ces dernières, au moins un premier ligand présentant une activité sélective pour les recepteurs RXRs et au moins un deuxième ligand présentant une activité sélective pour le récepteur RAR-α à titre de principes actifs. Il est bien entendu possible de mettre en oeuvre un ou plusieurs ligands présentant une activité spécifique vis à vis des récepteurs RXRs en association avec un ou plusieurs ligands présentant une activité spécifique vis à vis du récepteur RAR-α.

L'administration des compositions selon l'invention peut être effectuée par voie entérale, parentérale, topique ou oculaire. Toutefois, de préférence, ces dernières compositions sont conditionnées sous une forme convenant à une application par voie systémique.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les mélanges d'actifs conformes à l'invention sont généralement administrés à des doses journalières d'environ 0,01 mg/kg à 100 mg/Kg en poids corporel, et ceci à raison de 1 à 3 prises/jour.

Par voie topique, les compositions pharmaceutiques, qui sont donc plus particulièrement destinées au traitement de la peau ou des muqueuses, peuvent se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée des actifs. Ces compositions par voie topique peuvent par ailleurs se présenter soit sous forme anhydre, soit sous une forme aqueuse, selon l'indication clinique.

Les compositions à usage topique conformes à l'invention contiennent le ou les rétinoïdes spécifiques des RXRs à une concentration généralement comprise entre 0,001 % et 10 % en poids, de préférence entre 0,1 et 1 % en poids, par rapport au poids total de la composition, et le ou les rétinoïdes spécifiques de RAR-α à une concentration généralement comprise entre 0,001 % et 10 % en poids, de préférence entre 0,1 et 1 % en poids, par rapport au poids total de la composition.

Par voie oculaire, ce sont principalement des collyres.

Les compositions médicamenteuses selon l'invention peuvent bien entendu en outre contenir des additifs inertes ou même pharmacodynamiquement actifs ou des combinaisons de ces additifs, et notamment des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétokonazole ou les polyméthylène-4,5 isothiazolidones-3; des antibactériens, des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

Comme indiqué précédemment, le caractère sélectif ou non d'une substance donné vis à vis d'un ou plusieurs récepteurs nucléaires donnés peut être déterminé au moyen de tests classiques pour l'homme de l'art. Ces tests sont notamment décrits dans les références suivantes : (1) "Selective Synthetic Ligands for Nuclear Retinoic Acid Receptor Subtypes" *in* RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" *in* Pharmacol Skin, Basel, Karger, 1993, Volume. 5, pp 117-127 ; (3) "Selective Synthetic Ligands for Human Nuclear Retinoic Acid Receptors" *in* Skin Pharmacology, 1992, Vol. 5, pp 57-65 ; (4) "Identification of Synthetic Retinoids with Selectivity for Human Nuclear Retinoic Acid Receptor-γ" *in* Biochemical and Biophysical Research Communications, Vol. 186, N° 2, Juillet 1992, pp 977-983 (5) "Selective High Affinity RAR-α or RAR-β Retinoic Acid Receptor Ligands" *in* Mol. Pharmacol., Vol 40, pp 556-562.

A titre d'exemples de rétinoïdes sélectifs de RAR-α utilisables dans le cadre de la présente invention, on peut notamment citer:
- l'acide 4-[(5,6,7,8-tétrahydro-5,5,8,8-tétraméthyl-2-naphtyl) carboxamido] benzoïque (composé 1, utilisé ci-après dans les exemples).
- l'acide 4-[(5,6,7,8-tétrahydro-5,5,8,8-tétraméthyl-2-naphtyl) carbamoyl] benzoïque (composé 2).

Comme exemples de rétinoïdes sélectifs des RXRs convenant bien à l'invention, on peut ici citer plus particulièrement:
- l'acide 4-[(5,6,7,8-tétrahydro-5,5,8,8-tétraméthyl-2-naphtyl)carbonyléthylèneacétal]benzoïque (composé 4, utilisé ci-après dans les exemples).
- l'acide (E)-2-[2-(3,5,5,8,8-pentaméthyl-5,6,7,8-tétrahydro-2-naphtyl)-1-propényl]-4-thiophènecarboxylique (composé 5).
- l'acide 4-[(3,5,5,8,8-pentaméthyl-5,6,7,8-tétrahydro-2-naphtyl)carbonyl] benzoïque (composé 6).

Comme indiqué précédemment, les compositions selon l'invention peuvent être avantageusement utilisées aux fins de moduler *in vitro* et/ou *in vivo* la prolifération et/ou la différenciation des systèmes cellulaires de type HL-60. Ces cellules ont été décrites notamment dans les publications suivantes : (1) "Induction of Differentiation of the Human Promyelocytic Leukemia Cell Line (HL-60) by Retinoic Acid" *in* Proc. Natl. Acad. Sci. USA, 77, pp 2936-2940, et (2), " "Human Leukemic Models of Myelomonocytic Development : a review of the HL-60 and U937 cell lines" *in* J. Leucocyte Biology, 37, pp 407-422, et elles sont en outre disponibles, sous forme de cultures, auprès de la Collection Européenne de Culture de Cellules Animales, Division of Biologics, Porton Down, Salisbury SP4 OJ4, Angleterre.

Plus spécifiquement encore, les compositions conformes à l'invention trouvent une application thérapeutique privilégiée dans le traitement *in vivo* et/ou *in vitro* de la leucémie promyélocytaire aigüe.

On va maintenant donner, à titre nullement limitatif, plusieurs exemples destinés d'une part à démontrer les effets attachés à la présente invention, et, d'autre part, à illustrer diverses formulations concrètes conformes à l'invention.

### EXEMPLE 1

Cet exemple a pour but de mettre en évidence l'activité in vitro des associations synergétiques conformes à l'invention sur la prolifération et/ou la différentiation des cellules HL-60.

Les cellules HL-60 utilisées sont des cellules qui ont été obtenues directement auprès la Collection Européenne de Culture de Cellules Animales identifiée ci-avant.

Le protocole expérimental et les méthodes de détermination des activités (prolifération, différentiation), étaient les suivants :

### Culture des cellules:

Les cellules sont cultivées à 37°C dans une atmosphère humide en présence de 5% de CO₂ dans du milieu RPMI 1640 (Gibco) additionné de glutamine (40 mM) et de 10 % (v/v) de sérum de veau foetal (SVF).

### Traitement des cellules:

Les cellules HL-60 sont ensemencées à raison de 4 10⁵ cellules dans 2 ml de milieu dans du milieu RPMI 1640 (Gibco) additionné de tampon Hepes pH 7,3 (10 mM), de glutamine (40 mM), de transférrine (5mg/ml), d'insuline (5 mg/ml), du sélénium (5 ng/ml) et de 2% (v/v) de SVF dans des puits de cluster de 10 cm². Les cellules sont traitées dès l'ensemencement avec les rétinoïdes (composés 1, 3 ou 4 tels que définis précédemment dans la description) dilués dans le DMSO. La concentration finale de DMSO dans le milieu de culture n'excède pas 0,2 % (v/v). Après 4 jours de culture, les cellules sont récoltées pour la détermination de la prolifération et de la différenciation.

### Mesure de la prolifération:

La prolifération des cellules est déterminée au moyen d'un appareillage référencé "Cell proliferation kit II" (dosage XTT) mis en oeuvre selon les indications du fabriquant (Boehringer, ref. 1465 05). Elle est exprimée en unité d'absorbance à 495 nm. Plus l'absorbance mesurée est faible, et plus la prolifération des cellules HL-60 a été inhibée.

### Mesure de la différenciation:

La différenciation des cellules HL-60 en granulocytes ou monocytes est mesurée avec le test NBT (nitro blue tétrazolium) (T.R. Breitman, Growth and differentiation of human myeloid leukemia cell line HL-60. in: Methods in Enzymology, vol. 190, pp.118- 130, (ed. L. Packer) Academic Press Inc. New York (1990)). Ce test permet de déterminer le nombre de cellules différenciées par rapport au nombre total de cellules.

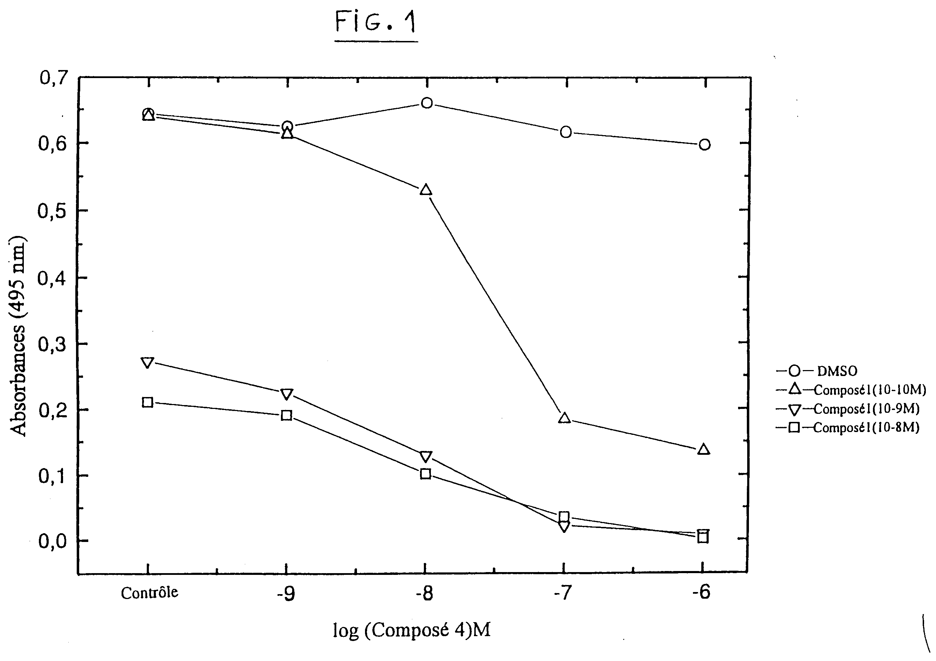

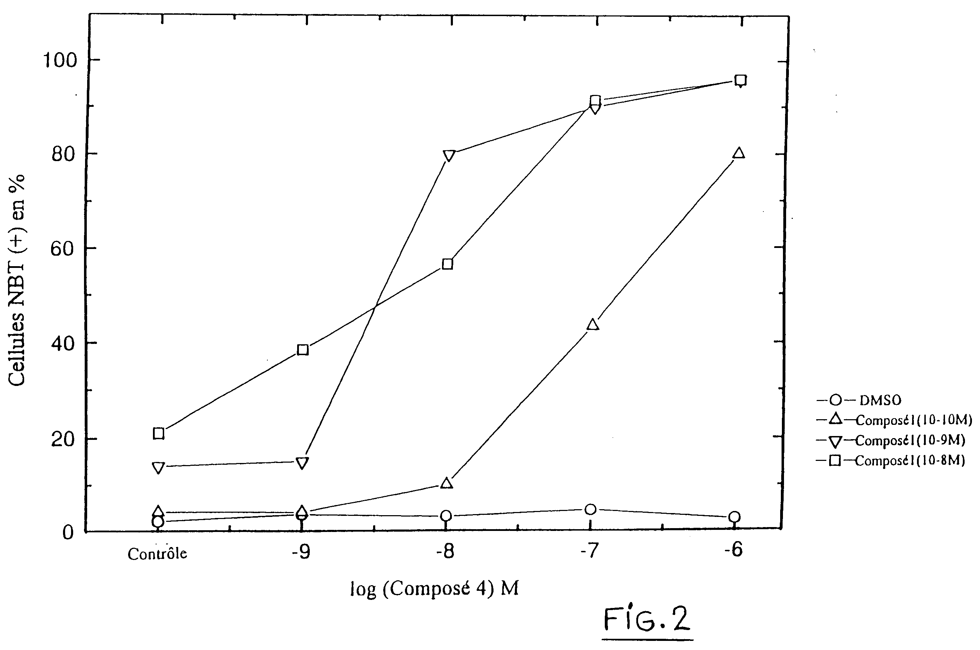

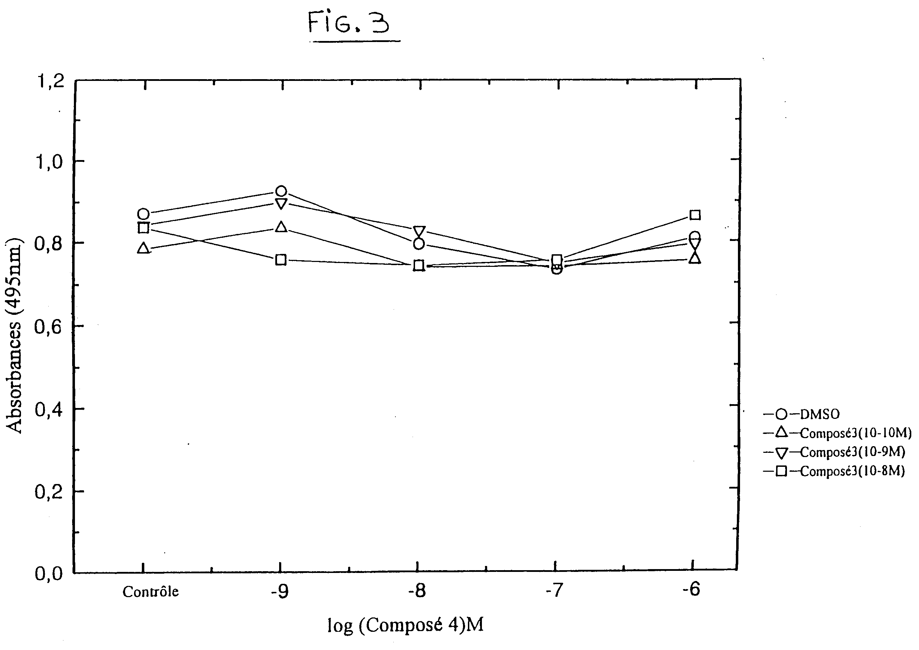

Tous les résultats obtenus sont rassemblés dans les figures 1 à 4. Ces figures quantifient l'évolution du taux d'inhibition de la prolifération des cellules HL-60 ou l'évolution du taux de cellules HL-60 différenciées en fonction des actifs utilisés d'une part et de leurs concentrations d'autre part. Les valeurs données au point de contrôle correspondent aux résultats trouvés lorsqu'on ne met en oeuvre aucun rétinoïde spécifique RXRs ; à l'inverse, la courbe DMSO (-0-) correspond aux résultats trouvés lorsqu'on met en oeuvre, à titre d'actif, uniquement un rétinoïde spécifique RXRs.

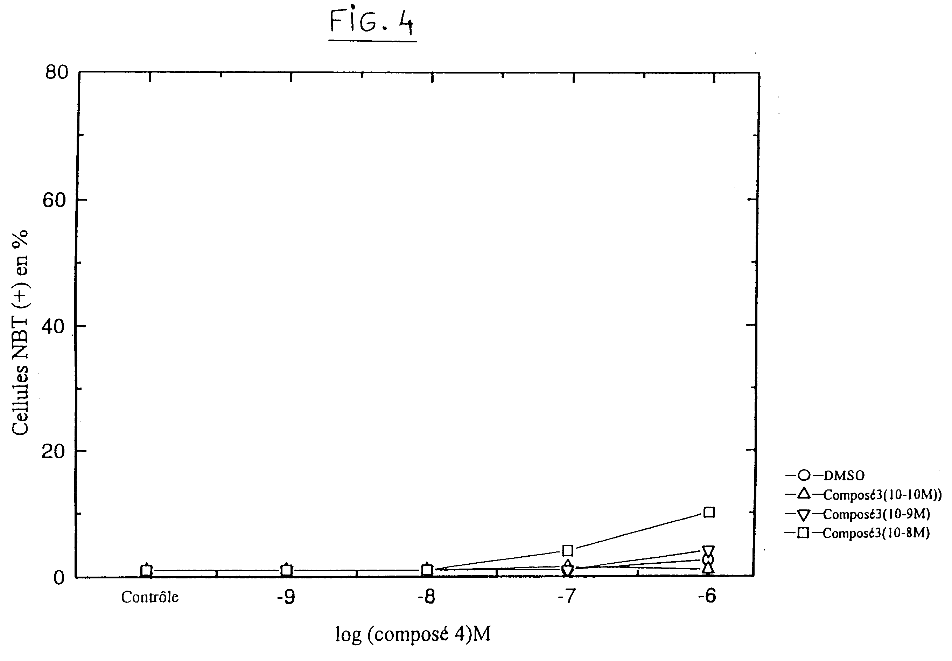

Ces figures montrent clairement que l'association d'un rétinoïde spécifique RAR-α (composé 1) et d'un rétinoïde spécifique des RXRs (composé 4) présente une très bonne activité sur la prolifération et la différenciation des cellules HL-60 (voir Figures 1 et 2) alors que l'association d'un rétinoïde spécifique de RAR-γ (composé 3) avec ce même rétinoïde spécifique des RXRs ne montre pas d'activité (voir Figures 3 et 4).

### EXEMPLE 2

Dans cet exemple, on a illustré diverses formulations concrètes à base des associations conformes à l'invention (les composés 1 et 4 sont ceux définis ci-avant dans la description).

### A- VOIE ORALE

| (a) Comprimé de 0,2 g | |
|---|---|
| - Composé 1 | 0,001 g |
| - Composé 4 | 0,001 g |
| - Amidon | 0,113g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 10 ml | |
|---|---|
| - Composé 1 | 0,05 g |
| - Composé 4 | 0,05 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10ml |

### B- VOIE TOPIQUE

| (a) Onguent | |
|---|---|
| - Composé 1 | 0,1 g |
| - Composé 4 | 0,1 g |
| - Myristate d'isopropyle | 81,520 g |
| - Huile de vaseline fluide | 9,100 g |
| - Silice ("Aérosil 200" vendue par DEGUSSA) | 9,180 g |

| (b) Crème Eau-dans-Huile non ionique | |
|---|---|
| - Composé 1 | 0,100 g |
| - Composé 4 | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (c) Lotion | |
|---|---|
| - Composé 1 | 0,100 g |
| - Composé 4 | 0,100 g |
| - Polyéthylène glycol (PEG 400) | 69,800 g |
| - Ethanol à 95% | 30,000 g |

| (d) Onguent hydrophobe | |
|---|---|
| - Composé 1 | 0,300 g |
| - Composé 4 | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) qsp | 100g |

| (e) Crème Huile-dans-Eau non ionique | |
|---|---|
| - Composé 1 | 0,500 g |
| - Composé 4 | 0,500 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile qsp | 100 g |

## Revendications

1. Composition, caractérisée par le fait qu'elle comprend, dans un support physiologiquement acceptable, au moins un premier ligand présentant une activité sélective pour les recepteurs RXRs et au moins un deuxième ligand présentant une activité sélective pour le récepteur RAR-α.

2. Composition selon la revendication précédente, caractérisée par le fait qu'il s'agit d'une composition conditionnée sous une forme convenant à un usage systémique.

3. Composition selon la revendication 1, caractérisée par le fait qu'il s'agit d'une composition conditionnée sous une forme convenant à un usage topique.

4. Composition selon la revendication 3, caractérisée par le fait que ledit premier ligand est présent à raison de 0,001 % à 10 % en poids, de préférence de 0,1 % à 1 % en poids, par rapport au poids total de la composition.

5. Composition selon la revendication 3 ou 4, caractérisée par le fait que ledit second ligand est présent à raison de 0,001 % à 10 % en poids, de préférence de 0,1 % à 1 % en poids, par rapport au poids total de la composition.

6. Composition selon l'une quelconque des revendications précédentes, caractérisée par le fait que ledit premier ligand est choisi parmi l'acide 4-[(5,6,7,8-tétrahydro-5,5,8,8-tétraméthyl-2-naphtyl)carbonyléthylèneacétal]benzoïque, l'acide (E)-2-[2-(3,5,5,8,8-pentaméthyl-5,6,7,8-tétrahydro-2-naphtyl)-1-propényl]-4-thiophènecarboxylique, l'acide 4-[(3,5,5,8,8-pentaméthyl-5,6,7,8-tétrahydro-2-naphtyl)carbonyl]benzoïque.

7. Composition selon l'une quelconque des revendications précédentes, caractérisée par le fait que ledit deuxième ligand à activité spécifique RAR-α est choisi parmi l'acide 4-[(5,6,7,8-tétrahydro-5,5,8,8-tétraméthyl-2-naphtyl)carboxamido]benzoïque et l'acide 4-[(5,6,7,8-tétrahydro-5,5,8,8-tétraméthyl-2-naphtyl) carbamoyl]benzoïque.

8. Composition selon l'une quelconque des revendications précédentes, pour une utilisation comme médicament.

9. Composition selon la revendication 8, pour une utilisation comme modulateur de la prolifération et/ou de la différenciation des systèmes cellulaires de type HL-60.

10. Composition selon l'une des revendications 8 ou 9, pour une utilisation destinée au traitement thérapeutique par voie systémique de la leucémie promyélocytaire aigüe.

11. Utilisation d'une composition telle que définie à l'une quelconque des revendications 1 à 7 pour la fabrication d'une composition pharmaceutique et/ou dermatologique destinée à moduler la prolifération et/ou de la différenciation des systèmes cellulaires de type HL-60.

12. Utilisation selon la revendication 11, destinée au traitement thérapeutique par voie systémique de la leucémie promyélocytaire aigüe.

## Claims

1. Composition, characterized in that it comprises, in a physiologically acceptable vehicle, at least one first ligand displaying selective activity for the RXR receptors and at least one second ligand displaying selective activity for the RAR-α receptor.

2. Composition according to the preceding claim, characterized in that it is a composition packaged in a form suitable for systemic use.

3. Composition according to Claim 1, characterized in that it is a composition packaged in a form suitable for topical use.

4. Composition according to Claim 3, characterized in that the said first ligand is present in the proportion of 0.001 % to 10 % by weight, and preferably 0.1 % to 1 % by weight, relative to the total weight of the composition.

5. Composition according to Claim 3 or 4, characterized in that the said second ligand is present in the proportion of 0.001 % to 10 % by weight, and preferably 0.1 % to 1 % by weight, relative to the total weight of the composition.

6. Composition according to any one of the preceding claims, characterized in that the said first ligand is chosen from 4-[(5,6,7,8-tetrahydro-5,5,8,8-tetramethyl-2-naphthyl)carbonyl]benzoic acid ethylene acetal, (E)-2-[2-(3,5,5,8,8-pentamethyl-5,6,7,8-tetrahydro-2-naphthyl)-1-propenyl]-4-thiophenecarboxylic acid and 4-[(3,5,5,8,8-pentamethyl-5,6,7,8-tetrahydro-2-naphthyl)carbonyl]benzoic acid.

7. Composition according to any one of the preceding claims, characterized in that the said second ligand having RAR-α-specific activity is chosen from 4-[(5,6,7,8-tetrahydro-5,5,8,8-tetramethyl-2-naphthyl)carboxamido]benzoic acid and 4-[(5,6,7,8-tetrahydro-5,5,8,8-tetramethyl-2-naphthyl)carbamoyl]benzoic acid.

8. Composition according to any one of the preceding claims, for use as a medicinal product.

9. Composition according to Claim 8, for use as a modulator of the proliferation and/or differentiation of HL-60 type cell systems.

10. Composition according to either of Claims 8 and 9, for use intended for the systemic therapeutic treatment of acute promyelocytic leukaemia.

11. Use of a composition as defined in any one of Claims 1 to 7, for the manufacture of a pharmaceutical and/or dermatological composition intended for modulating the proliferation and/or differentiation of HL-60 type cell systems.

12. Use according to Claim 11, intended for the systemic therapeutic treatment of acute promyelocytic leukaemia.

## Patentansprüche

1. Zusammensetzung, dadurch gekennzeichnet, daß sie in einem physiologisch akzeptablen Träger mindestens einen ersten Liganden, der eine selektive Wirksamkeit gegenüber RXR-Rezeptoren besitzt, und mindestens einem zweiten Liganden, der eine selektive Wirksamkeit gegenüber dem Rezeptor RAR-α besitzt, enthält.

2. Zusammensetzung nach dem vorhergehenden Anspruch, dadurch gekennzeichnet, daß es sich um eine Zusammensetzung handelt, die in einer für eine systemische Anwendung geeigneten Form formuliert ist.

3. Zusammensetzung nach Anspruch 1, dadurch gekennzeichnet, daß es sich um eine Zusammensetzung handelt, die in einer für eine topische Anwendung geeigneten Form formuliert ist.

4. Zusammensetzung nach Anspruch 3, dadurch gekennzeichnet, daß der erste Ligand in einem Mengenanteil von 0,001 bis 10 Gew.-% und vorzugsweise 0,1 bis 1 Gew.-%, bezogen auf das Gesamtgewicht der Zusammensetzung, vorliegt.

5. Zusammensetzung nach Anspruch 3 oder 4, dadurch gekennzeichnet, daß der zweite Ligand in einem Mengenanteil von 0,001 bis 10 Gew.-% und vorzugsweise 0,1 bis 1 Gew.-%, bezogen auf das Gesamtgewicht der Zusammensetzung, vorliegt.

6. Zusammensetzung nach einem der vorhergehenden Ansprüche, dadurch gekennzeichnet, daß der erste Ligand unter 4-[(5,6,7,8-Tetrahydro-5,5,8,8-tetramethyl-2-naphthyl)-carbonylethylenacetal]-benzoesäure, (E)-2-[2-(3,5,5,8,8-Pentamethyl-5,6,7,8-tetrahydro-2-naphthyl)-1-propenyl]-4-thiophencarbonsäure und 4-[3,5,5,8,8-Pentamethyl-5,6,7,8-tetrahydro-2-naphthyl)-carbonyl]-benzoesäure ausgewählt ist.

7. Zusammensetzung nach einem der vorhergehenden Ansprüche, dadurch gekennzeichnet, daß der zweite Ligand mit der spezifischen Wirksamkeit gegenüber dem Rezeptor RAR-α unter 4-[(5,6,7,8-Tetrahydro-5,5,8,8-tetramethyl-2-naphthyl)-carboxamido]-benzoesäure und 4-[5,6,7,8-Tetrahydro-5,5,8,8-tetramethyl-2-naphthyl)-carbamoyl]-benzoesäure ausgewählt ist.

8. Zusammensetzung nach einem der vorhergehenden Ansprüche zur Verwendung als Arzneimittel.

9. Zusammensetzung nach Anspruch 8 zur Verwendung als Modulator der Proliferation und/oder der Differenzierung von Zellsystemen vom Typ HL-60.

10. Zusammensetzung nach Anspruch 8 oder 9 zur Verwendung zur systemischen therapeutischen Behandlung der akuten Promyelocytenleukämie.

11. Verwendung einer Zusammensetzung nach einem der Ansprüche 1 bis 7 zur Herstellung einer pharmazeutischen und/oder dermatologischen Zusammensetzung zur Modulation der Proliferation und/oder der Differenzierung von Zellsystemen vom Typ HL-60.

12. Verwendung nach Anspruch 11 zur systemischen therapeutischen Behandlung der akuten Promyelocytenleukämie.
